# EUROPÄISCHE PATENTSCHRIFT

(11) **EP 2 877 443 B1**
(45) Veröffentlichungstag und Bekanntmachung des Hinweises auf die Patenterteilung: **11.05.2016**
(21) Anmeldenummer: 13742009.7
(22) Anmeldetag: 23.07.2013
(51) Int. Cl.: C07C 201/08

(54) **VERFAHREN ZUR HERSTELLUNG VON NITROBENZOL DURCH ADIABATE NITRIERUNG**
METHOD FOR MANUFACTURING NITROBENZOL THROUGH ADIABATIC NITRATION
PROCEDE DESTINE À LA FABRICATION DE BENZOL NITRIQUE PAR NITRIFICATION D'ADIABATE

(30) Priorität: 27.07.2012 EP 12178160
(43) Veröffentlichungstag der Anmeldung: 03.06.2015
(73) Patentinhaber: Covestro Deutschland AG, 51373 Leverkusen (DE)
(72) Erfinder: KNAUF, Thomas, 41542 Dormagen (DE); RACOES, Alexandre, 47805 Krefeld (DE); DOHMEN, Wolfgang, 47229 Duisburg (DE); MAIRATA, Antoni, 40549 Düsseldorf (DE)
(74) Vertreter: Levpat
(86) Internationale Anmeldenummer: PCT/EP2013/065502
(87) Internationale Veröffentlichungsnummer: WO 2014/016289

(56) Entgegenhaltungen:
- EP-A1- 2 246 320
- DE-A1-102009 005 324

## Beschreibung

Gegenstand der Erfindung ist ein adiabates Verfahren zur Herstellung von Nitrobenzol durch Nitrierung von Benzol mit Mischungen aus Schwefel- und Salpetersäure unter Einsatz eines stöchiometrischen Überschusses an Benzol unter Wiederverwendung nicht umgesetzten Benzols, bei dem der Gehalt an aliphatischen organischen Verbindungen im Einsatzbenzol durch gezielte Ausschleusung aliphatischer organischer Verbindungen an mindestens einer Stelle des Prozesses auf einen Gehalt von kleiner als 1,5 Massen-%, bezogen auf die Gesamtmasse des Einsatzbenzols, beschränkt wird.

Die vorliegende Erfindung betrifft ein kontinuierliches Verfahren zur Herstellung von Nitrobenzol durch adiabate Nitrierung von Benzol durch ein Gemisch von Schwefel- und Salpetersäure (sog. Mischsäure). Ein solches Verfahren wurde erstmals in US 2,256,999 beansprucht und ist in moderneren Ausführungsformen in US 4,091,042, US 5,313,009 und US 5,763,697 beschrieben.

Den beschriebenen adiabaten Verfahren ist gemein, dass die Ausgangsstoffe Benzol und Salpetersäure in einem großen Überschuss an Schwefelsäure zur Reaktion gebracht werden, welche die frei werdende Reaktionswärme und das bei der Reaktion gebildete Wasser aufnimmt.

Die Reaktionsführung erfolgt im Allgemeinen so, dass die Salpetersäure und Schwefelsäure zur sogenannten Nitriersäure (auch Mischsäure genannt) vereint werden. Benzol wird in diese Nitriersäure hinein dosiert. Die Reaktionsprodukte sind im Wesentlichen Wasser und Nitrobenzol. In der Nitrierreaktion wird Benzol bezogen auf die molare Salpetersäuremenge mindestens in stöchiometrischer Menge, aber bevorzugt in 2%igem bis 10%igem Überschuss eingesetzt. Das in den Reaktionsapparaten gebildete und im Phasentrennapparat von der Säurephase abgetrennte Rohnitrobenzol wird gemäß dem Stand der Technik einer Wäsche und einer destillativen Aufarbeitung unterzogen, wie beispielsweise in EP 1 816 117 B1 (Seite 2, Zeile 26 bis 42), US 4,091,042 (siehe oben) oder US 5,763,697 (siehe oben) beschrieben. Charakteristisch für diese Aufarbeitung ist, dass nicht umgesetztes überschüssiges Benzol nach der Wäsche von Nitrobenzol in einer abschließenden Destillation abgetrennt wird und als Rückbenzol, das auch niedrig siedende, nicht-aromatische organische Verbindungen (sog. Leichtsieder) enthält (siehe DE 10 2009 005 324 A1), wieder in der Nitrierreaktion eingesetzt wird. Die Behandlung des Abgases aus der adiabaten Nitrierreaktion wird in EP 0 976 718 B1 beschrieben. Das Abgas aus Säurekreislauf und fertigem Rohnitrobenzol wird abgezogen, vereint und über einen NOx-Absorber geschickt, um verdünnte Salpetersäure zurückzugewinnen, die in die Reaktion zurückgefahren wird. Die als Kreislaufsäure bezeichnete Schwefelsäure wird in einem Blitzverdampfer aufkonzentriert und weitestgehend von Organika befreit.

Es verbleiben hochsiedende Organika wie z. B. Nitrobenzol, Dinitrobenzol und Nitrophenole in Spuren in der Kreislaufsäure und werden somit auch zur Reaktion zurückgefahren.

Die Güte eines adiabaten Verfahrens zur Nitrierung aromatischer Kohlenwasserstoffe ist einerseits definiert durch den Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Das Bestreben bei der Herstellung von Nitrobenzol ist es, den Gehalt an Dinitrobenzol und an Nitrophenolen, insbesondere des als brisant einzustufenden Trinitrophenols (Pikrinsäure), zu minimieren.

Andererseits ist die Güte eines adiabaten Verfahrens dadurch definiert, Nitrobenzol mit möglichst geringem Einsatz an Energie herzustellen. Dies wird unter anderem dadurch gewährleistet, dass die adiabate Wärme der Reaktion zur Aufkonzentrierung der Schwefelsäure genutzt wird, oder dass der in der Reaktion benötigte stöchiometrische Benzolüberschuss in Bezug auf Salpetersäure möglichst gering gehalten wird, um die Wäschen des Rohnitrobenzols und die abschließende Destillation desselbigen energetisch nicht unnötig zu belasten.

Um Nitrobenzol mit besonders hohen Selektivitäten zu erhalten, wurde die Art der zu verwendenden Mischsäure detailliert festgelegt (EP 0 373 966 B1, EP 0 436 443 B1 und EP 0 771 783 B1), sowie darauf hingewiesen, dass der Gehalt an Nebenprodukten durch die Höhe der Maximaltemperatur bestimmt wird (EP 0 436 443 B1, Spalte 15, Z. 22 bis 25). Auch ist bekannt, dass ein hoher Anfangsumsatz vorteilhaft ist für eine hohe Selektivität und dass diese dann erreicht wird, wenn zu Beginn der Reaktion eine optimale Durchmischung herbeigeführt wird (EP 0 771 783 B1, Absatz [0012 bis 0014]).

Zu hervorragenden Selektivitäten gelangt man, wenn die Reaktionsstarttemperatur sehr niedrig gewählt wird (WO2010/051616 A1), was aber gleichbedeutend mit einer mehrfach verlängerten Reaktionszeit ist. Eine hohe Raum-Zeit-Ausbeute ist für die industrielle Anwendung eines Verfahrens von Vorteil, da dadurch kompakte Reaktionseinrichtungen gebaut werden können, die sich durch ein geringes Investitionsvolumen pro Kapazität auszeichnen. Daher ist diese Vorgehensweise kontraproduktiv.

Bezüglich der Qualität des Einsatzstoffes Benzol auf die adiabate Herstellung von Nitrobenzol beschreibt EP 2 246 320 A1, dass handelsübliches Benzol je nach Quelle mehr oder weniger stark verunreinigt sein kann. Typische Verunreinigungen sind andere Aromaten, insbesondere Toluol und Xylol, die in Benzol gängiger Reinheit bis zu jeweils 0,05 Massen-% enthalten sein können. Andere für Benzol typische Verunreinigungen sind nichtaromatische organische Verbindungen, die in Summe bis zu 0,07 Massen-% ausmachen können. Insbesondere sind hier Cyclohexan (bis zu 0,03 Massen-%) und Methylcyclohexan (bis zu 0,02 Massen-%) genannt. Die vorstehend beschriebenen Verunreinigungen stören die nachfolgenden Schritte in der MDI-Prozesskette (MDI = Di- und Polyisocyanate der Diphenylmethanreihe) in den genannten Konzentrationen entweder überhaupt nicht oder nur geringfügig, beispielsweise durch eine minimale Erschwerung der Abwasser- und Abluftaufbereitung im Nitrobenzolverfahren durch nichtaromatische Organika im Benzol. Eine aufwändige Reinigung des Benzols für den Einsatz in der MDI-Prozesskette wird daher für unverhältnismäßig erachtet und kann unterbleiben. In EP 2 246 320 A1 wird nicht auf die nichtaromatischen organischen Verbindungen im nach erfolgter Reaktion vom Rohprodukt abgetrennten und in die Nitrierung zurückgeführten Benzol (sog. "Rückbenzol") eingegangen.

DE 10 2009 005 324 A1 offenbart, dass technisches Benzol üblicherweise einen Anteil von leichtsiedenden nichtaromatischen organischen Verbindungen (Leichtsieder) von 0,01 % bis 0,5 % hat. In den gängigen Verfahren der Benzolnitrierung wird jedoch nicht technisches Benzol als solches eingesetzt, sondern eine Mischung aus Rückbenzol und technischem Benzol, sodass der Gehalt an Leichtsiedern im tatsächlich eingesetzten Benzol beträchtlich höher sein kann als in handelsüblichem technischem Benzol. DE 10 2009 005 324 A1 offenbart beispielhaft einen Wert von 5 % (Abschnitt [0007]). Gemäß der Lehre dieser Schrift ist ein solch hoher Leichtsiederanteil in der eigentlichen Nitrierung noch nicht nachteilig. DE 10 2009 005 324 A1 geht nur auf Probleme bei der sich anschließenden Phasentrennung ein (Abschnitt [0008]). Zur Lösung dieser Probleme wird ein spezielles Phasentrennverfahren unter Einsatz eines Druckhalte-Syphons vorgeschlagen.

EP 2 155 655 B1 geht nur auf aromatische Verunreinigungen (Alkyl-substituierte Aromaten) im Benzol ein.

DE-AS-1 129 466 beschreibt ein Verfahren zum Mononitrieren von technischem Benzol, Xylol und Toluol, die die üblichen Gehalte an aliphatischen Kohlenwasserstoffen aufweisen, bei dem der in der Destillation des Nitroaromaten anfallende Vorlauf an nicht umgesetztem Aromaten, der reich an aliphatischen Verunreinigungen ist, mit frischem Aromaten vermischt, der Nitrierung zugeführt und der erneut anfallende Vorlauf beliebig oft im Kreis geführt wird. (Im Falle von Benzol als Ausgangstoff entspricht der Aromatenvorlauf dieser Schrift dem oben erwähnten Rückbenzol.) Der Fachmann entnimmt dieser Schrift mithin die technische Lehre, dass ein erhöhter Anteil an aliphatischen Verunreinigungen im einzusetzenden Aromaten die Nitrierung nicht stört.

EP 0 976 718 A2 offenbart ein Verfahren bei dem das Abgas in einem NOx-Absorber behandelt und dann verbrannt wird. Die dadurch entstehenden Benzolverluste und die auf diesem Wege ausgeschleusten Leichtsieder werden nicht angesprochen.

Den beschriebenen Verfahren des Standes der Technik gelingt es zwar, ein Nitrobenzol herzustellen, das einen geringen Gehalt an Nebenprodukten aufweist, also nur von etwa 100 ppm bis 300 ppm an Dinitrobenzol und 1500 ppm bis 2500 ppm an Nitrophenolen enthält, wobei Pikrinsäure einen Anteil von 10 Massen-% bis 50 Massen-% der Nitrophenole annehmen kann. Die Verfahren zeichnen sich auch durch eine hohe Raum-/Zeitausbeute aus.

Neben der Reinheit des Rohnitrobenzols ist von entscheidender Bedeutung für die industrielle Produktion, dass die Herstellung des Nitroaromaten in möglichst kompakten Reaktionseinrichtungen unter energetisch günstigen Bedingungen (wie geringem Benzolüberschuss) durchgeführt werden kann. Ebenfalls wünschenswert sind möglichst günstige Rohstoffe, die jedoch gegebenenfalls unerwünschte Nebenkomponenten enthalten.

Verunreinigungen im frisch zugeführten Benzol und/oder im Rückbenzol erniedrigen die insgesamt verfügbare Konzentration an Benzol. Hierdurch wird die Reaktion verlangsamt. Die verringerte Benzolkonzentration kann zur Folge haben, dass eine zu große Menge an Salpetersäure eingesetzt wird. Dies wiederum erhöht die Menge an unerwünschten mehrfach nitrierten Produkten und NOx-Gasen. Letzteres hat die Bildung weiterer Nebenprodukte zur Folge. In diesem Sinne kritische Verunreinigungen sind insbesondere aliphatische organische Verbindungen (Leichtsieder, siehe oben). Diese können während der Reaktion gemeinsam mit NOx-Gasen ausgasen und dadurch weitere Nachteile hervorrufen wie bspw. eine schlechtere Durchmischung der Reaktanden und ein reduziertes Reaktionsvolumen. Es bestand daher ein Bedarf an einer Methode den Gehalt an aliphatischen organischen Verbindungen im tatsächlich eingesetzten Benzol eines adiabaten Nitrierprozesses zu minimieren oder im Idealfall ganz zu verhindern.

Dem vorstehend Gesagten Rechnung tragend befasst sich die vorliegende Erfindung damit, den negativen Einfluss aliphatischer organischer Verbindungen zu minimieren. Zu diesem Zweck wurden zwei grundsätzliche Varianten entwickelt:
In der **ersten Variante** ist ein Gegenstand der vorliegenden Erfindung ein kontinuierlich betriebenes adiabates Verfahren zur Herstellung von Nitrobenzol durch Nitrierung von Benzol in einer Nitrieranlage, bei dem
   a) ein Benzol-haltiger Strom (a.1), der mindestens 90 Massen-%, bevorzugt mindestens 95 Massen-%, und besonders bevorzugt mindestens 99 Massen-% Benzol, jeweils bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol in einem stöchiometrischen Überschuss bezogen auf Salpetersäure (a.3) von bevorzugt 2,0 % bis 20 %, besonders bevorzugt von 5,0 % bis 10 % der Theorie eingesetzt wird,
   b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
   c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, und wobei die Phase (c.1) teilweise bis vollständig in Schritt a) zurückgeführt und als Bestandteil von (a.2) eingesetzt wird,
   d) die in Schritt b) erhaltene organische Phase (b.2) zu Rein-Nitrobenzol (d.1) aufgearbeitet wird, bevorzugt durch Waschen mit wässrigen Medien und sich daran anschließender Rektifikation, wobei ein Benzol-haltiger Strom (d.2) gewonnen wird (das sog. "Rückbenzol"), der bevorzugt 40,0 Massen-% bis 99,9 Massen-%, besonders bevorzugt 60,0 Massen-% bis 99,9 Massen-%, ganz besonders bevorzugt 80,0 Massen-% bis 99,9 Massen-% Benzol, außerordentlich ganz besonders bevorzugt von 90,0 Massen-% bis 99,9999 Massen-% Benzol, jeweils bezogen auf die Gesamtmasse von (d.2), umfasst, und der teilweise bis vollständig als Bestandteil von (a.1) in der Nitrieranlage aus Schritt a) verwendet wird,
und bei dem
aliphatische organische Verbindungen an mindestens einer Stelle gezielt so aus dem Prozess ausgeschleust werden, dass dem Reaktor nur ein solcher Benzol-haltiger Strom (a.1) zugeführt wird, der einen Gehalt an aliphatischen organischen Verbindungen von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse von (a.1), aufweist.

In der **zweiten Variante** ist ein Gegenstand der vorliegenden Erfindung ein adiabates Verfahren zur Herstellung von Nitrobenzol durch Nitrierung von Benzol in zwei hintereinander geschalteten Nitrieranlagen (100, 200) mit mindestens je einem Reaktor (1, 1*), wobei
a) im Reaktor (1) der ersten, kontinuierlich betriebenen, Nitrieranlage (100) ausschließlich Benzol (a.1.1), das einen Gehalt an aliphatischen organischen Verbindungen von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse von (a.1.1), aufweist, mit einem Gemisch aus Schwefelsäure (a.2.1) und Salpetersäure (a.3.1) unter adiabaten Bedingungen umgesetzt wird, wobei Benzol in einem stöchiometrischen Überschuss bezogen auf Salpetersäure (a.3.1) von bevorzugt 2,0 % bis 20 %, besonders bevorzugt von 5,0 % bis 10 % der Theorie, eingesetzt wird;
b) das in Schritt a) erhaltene Verfahrensprodukt in einem Phasentrennapparat (2) in eine wässrige, Schwefelsäure umfassende Phase (b.1.1) und eine organische, Nitrobenzol umfassende Phase (b.2.1) getrennt wird,
c) die in Schritt b) erhaltene wässrige Phase (b.1.1) durch Verdampfung von Wasser zu einer wässrigen Phase (c.1.1) mit gegenüber (b.1.1) erhöhter Schwefelsäurekonzentration aufkonzentriert wird, und wobei die Phase (c.1.1) teilweise bis vollständig in Schritt a) zurückgeführt und als Bestandteil von (a.2.1) eingesetzt wird,
d) die in Schritt b) erhaltene organische Phase (b.2.1) zu Rein-Nitrobenzol (d.1.1) aufgearbeitet wird, wobei ein Benzol-haltiger Strom (d.2.1) gewonnen wird, der bevorzugt 40,0 Massen-% bis 99,9 Massen-%, besonders bevorzugt 60,0 Massen-% bis 99,9 Massen-%, ganz besonders bevorzugt 80,0 Massen-% bis 99,9 Massen-% Benzol, außerordentlich ganz besonders bevorzugt von 90,0 Massen-% bis 99,9999 Massen-% Benzol, jeweils bezogen auf die Gesamtmasse von (d.2.1), umfasst
und bei dem der Benzol-haltige Strom (d.2.1) in der zweiten, kontinuierlich oder diskontinuierlich betriebenen, Nitrieranlage (200) in einem Reaktor (1*) mit einem Gemisch aus Schwefelsäure (a.2.2) und Salpetersäure (a.3.2) unter adiabaten Bedingungen zu Nitrobenzol umgesetzt wird, wobei Benzol in einem stöchiometrischen Überschuss bezogen auf Salpetersäure (a.3.2) von bevorzugt 2,0 % bis 20 %, besonders bevorzugt von 5,0 % bis 10 % der Theorie, eingesetzt wird, und wobei das so gebildete Nitrobenzol anschließend zu Rein-Nitrobenzol aufgearbeitet wird.

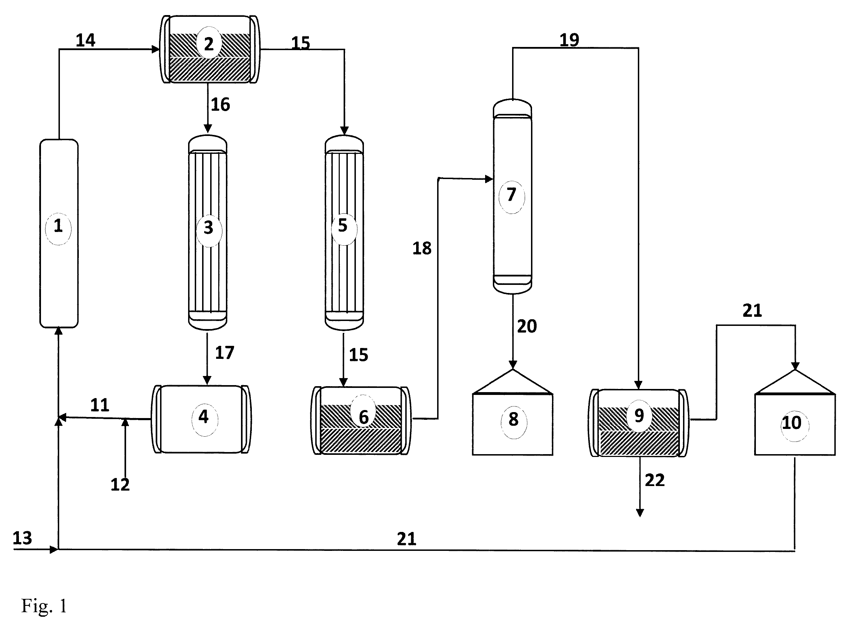

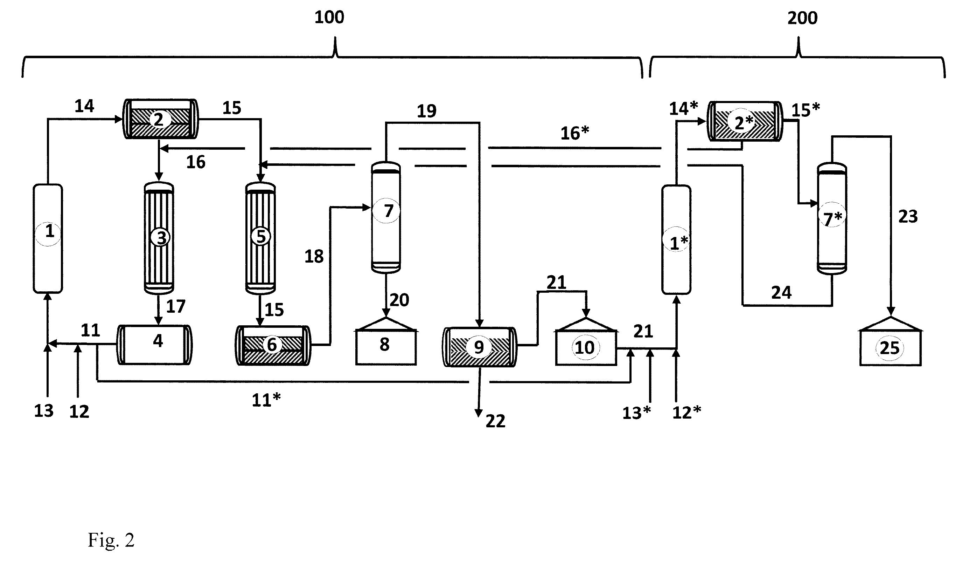

Eine *Nitrieranlage* umfasst erfindungsgemäß mindestens einen Nitrierreaktor und mindestens einen Phasentrennapparat sowie die zu deren Betrieb erforderlichen Peripheriegeräte wie Leitungen, Pumpen, ggf. Wärmeausstauscher etc. Die für die weitere Verarbeitung der nach der Phasentrennung des Rohprodukts der Nitrierung erhaltenen organischen, Nitrobenzol umfassenden Phase und der wässrigen, Schwefelsäure umfassenden Phase erforderlichen Apparate (Verdampfungsapparat und Aufarbeitungsvonichtungen wie Wäscher und eine Destillationskolonne) können in der Ausführungsform mit zwei hintereinander geschalteten Nitrieranlagen (100, 200) von beiden Nitrieranlagen (100, 200) gemeinsam benutzt werden (vgl. auch Fig. 2). Ist nur eine Nitrieranlage vorhanden (vgl. auch Fig. 1), sind diese Apparate natürlich auch Bestandteil derselben.

Der *auf Salpetersäure bezogene Benzolüberschuss* von 2,0 % bis 20 %, bevorzugt von 5,0 % bis 10 % der Theorie, bezieht sich auf das molare Verhältnis von Benzol und Salpetersäure. Theoretisch reagiert ein Mol Salpetersäure mit einem Mol Benzol zu einem Mol Nitrobenzol.

*Aliphatische organischen Verwindungen* im Sinne der vorliegenden Erfindung umfassen bevorzugt Cyclohexan, Heptan, Methylcyclohexan, Bicycloheptan, Isomere des Dimethylcyclopentans, Ethylcyclopentan, Pentan, Cyclopentan und 2-Methylhexan. Erfindungsgemäß muss der Gehalt an aliphatischen organischen Verbindungen im Benzol-haltigen Strom (a.1) (dem sog. "Einsatzbenzol") kontrolliert werden. Zu diesem Zweck sind analytische Messungen erforderlich. Gemessen werden das Frischbenzol im Tank, das Einsatzbenzol und das Rückbenzol (d.2), und zwar bevorzugt durch Probenahme an den entsprechenden Stellen und Analyse der Proben mittels Gaschromatographie. Andere Bestimmungsmethoden (z. B. spektroskopische Methoden), ggf. auch online oder inline, können, wenn auch nicht bevorzugt, grundsätzlich ebenfalls angewandt werden. Maßgeblich für die erfindungsgemäße Obergrenze des Gehalts an organischen aliphatischen Verbindungen ist jedoch der durch Gaschromatographie ermittelte Wert. Die Analyse des Stroms (d.2) erfolgt in einer bevorzugten Ausführungsform, nämlich der Aufarbeitung mittels Rektifikation, am Kopf der Rektifikationskolonne.

Das Wort "*ein*" ist im Rahmen dieser Erfindung im Zusammenhang mit zählbaren Größen nur dann als Zahlwort zu verstehen, wenn dies ausdrücklich gesagt wird. Bspw. schließt der Ausdruck "*ein Reaktors"* die Möglichkeit des Vorhandenseins mehrerer (in Reihe oder parallel geschalteter) Reaktoren innerhalb einer Nitrieranlage nicht aus.

Erfindungswesentlich ist nun, dass der Gehalt an aliphatischen organischen Verbindungen im Benzol-haltigen Strom (a.1) bzw. (a.1.1) (dem sog. "Einsatzbenzol" der Nitrieranlage bzw. der ersten von zwei hintereinander geschalteten Nitrieranlagen) den zuvor genannten Konzentrationen entspricht. Dieses Ziel wird in beiden Varianten letztlich durch gezielte Ausschleusung der aliphatischen organischen Verbindungen an mindestens einer Stelle des Prozesses erreicht.

In der ersten Variante der vorliegenden Erfindung erfolgt diese Ausschleusung, die kontinuierlich oder diskontinuierlich durchgeführt werden kann, derart, dass der in Schritt a) derselben Nitrieranlage zurückgeführte Benzol-haltige Strom (d.2) eine solche Konzentration an aliphatischen organischen Verbindungen aufweist, dass das Einsatzbenzol (a.1) den erfindungsgemäßen Anforderungen genügt. Details zur Ausgestaltung der gezielten Ausschleusung werden weiter unten beschrieben.

In der zweiten Variante der vorliegenden Erfindung verbleiben die aliphatischen organischen Verbindungen überwiegend bis vollständig im Benzol-haltigen Strom (d.2.1) der ersten Nitrieranlage (100) und werden mit diesem aus der ersten Nitrieranlage (100) ausgeschleust. Dieser Benzol-haltige Strom (d.2.1) dient als - bevorzugt einziger - Bestandteil des Einsatzbenzols (a.1.2) der zweiten Nitrieranlage (200). Da in der zweiten Nitrieranlage (200) im Wesentlichen nur das Überschussbenzol der ersten Nitrieranlage (100) verarbeitet wird, fällt diese wesentlich kleiner aus als die erste Nitrieranlage (100). Hier fallen die Nachteile eines ggf. erhöhten Gehalts an aliphatischen organischen Verbindungen im Einsatzbenzol weit weniger ins Gewicht. Diese Vorgehensweise erlaubt es, als Einsatzbenzol (a.1.1) der ersten Nitrieranlage (100) ausschließlich (d. h., ohne Beimischungen rezyklierten "Rückbenzols") kommerziell erhältliches Benzol zu verwenden (sofern dieses den erfindungsgemäßen Anforderungen im Hinblick auf den maximalen Gehalt an aliphatischen organischen Verbindungen genügt, was jedoch in der Regel der Fall ist); eine Vorgehensweise, die bei den aus dem Stand der Technik bekannten Verfahren unwirtschaftlich wäre.

Nachstehend werden für beide Varianten Ausführungsformen näher beschrieben. Verschiedene Ausführungsformen sind dabei beliebig miteinander kombinierbar, solange sich für den Fachmann aus dem Kontext nicht eindeutig das Gegenteil ergibt.

### Erste Variante:

In einer Ausführungsform des erfindungsgemäßen Verfahrens erfolgt die gezielte Ausschleusung der aliphatischen organischen Verbindungen in Schritt b). Zu diesem Zweck wird die Phasentrennung in Schritt b) unter solchen Bedingungen durchgeführt, dass die aliphatischen organischen Verbindungen überwiegend in der Gasphase des eingesetzten Phasentrennapparates verbleiben und mit dieser ausgeschleust werden. Der relativ zum Atmosphärendruck in der Gasphase des Phasentrennapparats gemessene Druck ("Überdruck") wird daher bevorzugt auf einen Wert von 20 mbar bis 200 mbar, ganz bevorzugt von 30 mbar bis 100 mbar eingestellt, und die Temperatur im Phasentrennapparat wird bevorzugt auf einen Wert von 100 °C bis 140°C, besonders bevorzugt von 120 °C bis 140 °C, eingestellt. Diese Gasphase (nachfolgend Leichtsiederstrom) wird dann über eine an den Gasraum des Phasentrennapparats angeschlossene Abgasleitung aus dem Prozess ausgeschleust und weiter behandelt. Insbesondere aufgrund der Möglichkeit der Anwesenheit von nitrosen Gasen (Stickoxiden) neben den aliphatischen organischen Verbindungen ist es bevorzugt, ein Inertgas, bevorzugt Stickstoff, in den Phasentrennapparat einzuspeisen, um dadurch die gasförmigen Stickoxide effektiv mit dem Leichtsiederstrom ausschleusen zu können. Gegenstand der Erfindung ist daher insbesondere auch ein Verfahren, bei dem zur Unterstützung der Ausschleusung der aliphatischen organischen Verbindungen und ggf. Stickoxiden ein Inertgas in den Phasentrennapparat eingespeist wird, mit dem zusammen aliphatische organische Verbindungen und ggf. Stickoxide über eine an den Gasraum des Phasentrennapparats angeschlossene Abgasleitung ausgeschleust werden.

Die Art der Weiterbehandlung des Leichtsiederstroms ist abhängig von seiner Zusammensetzung, d. h., insbesondere vom Anteil des mitgerissenen Benzols. Bei geringem Benzolgehalt wird der Leichtsiederstrom bevorzugt verbrannt. Ist der Benzolgehalt des Leichtsiederstroms hinreichend hoch, kann jedoch eine Wiedergewinnung oder Nutzbarmachung des darin enthaltenen Benzols wirtschaftlich sinnvoller sein. Ein Gegenstand der vorliegenden Erfindung ist daher auch ein Verfahren, bei dem ausgeschleuste aliphatische organische Verbindungen teilweise bis vollständig zur Rückgewinnung von mit den aliphatischen organischen Verbindungen gemeinsam ausgeschleustem Benzol destillativ aufgearbeitet werden. Der abgetrennte Leichtsiederstrom wird zu diesem Zweck bevorzugt durch ein- oder mehrstufige Kondensation verflüssigt und einem Phasentrennapparat zugeführt, um gleichfalls kondensiertes Wasser abzutrennen. Bevorzugt werden die aliphatischen organischen Verbindungen aus der so erhaltenen organischen Phase abdestilliert, wobei Benzol als Sumpfstrom der Destillation gewonnen wird. Das zurückgewonnene Benzol wird bevorzugt dem Strom (a.1) zugeschlagen. Diese Vorgehensweise ist dann bevorzugt, wenn der Benzol-Gehalt im Leichtsiederstrom größer als 50,0 Massen-%, bevorzugt größer als 70,0 Massen-%, jeweils bezogen auf die Gesamtmasse des Leichtsiederstroms, ist. Die abgetrennten aliphatischen organischen Verbindungen werden bevorzugt verbrannt oder der petrochemischen Industrie wieder zur Verfügung gestellt. Diese Variante hat den Vorteil, dass die abgetrennten aliphatischen organischen Verbindungen die Aufarbeitung in Schritt d) nicht durchlaufen. Insbesondere kann es in der bevorzugten Aufarbeitungsvariante durch Waschen mit wässrigen Medien und anschließender Rektifikation bei Vorhandensein von zu großen Mengen an aliphatischen organischen Verbindungen zu Phasentrennproblemen kommen, und zwar insbesondere in der Phasentrennung der sog. "neutralen Wäsche" (siehe unten bei "Schritt d(iii)" für Details). So wurde beobachtet, dass bei zu hohem Gehalt an aliphatischen organischen Verbindungen die Phasentrennzeit stark ansteigt.

In einer weiteren Ausführungsform erfolgt die gezielte Ausschleusung der aliphatischen organischen Verbindungen in Schritt d). Dabei wird der Brüdenstrom der in Schritt d) bevorzugt eingesetzten Benzol-Rektifikationskolonne umfassend Benzol, aliphatische organische Verbindungen und Restwasser aus demNitrobenzol abdestilliert und dabei das Nitrobenzol-Produkt (d.1) als Sumpfstrom erhalten. Die Mischung aus Benzol und aliphatischen organischen Verbindungen wird von dem Restwasser getrennt. Das abgetrennte Restwasser wird zur Wäsche zurückgefahren. Ein Teil des Gemisches aus Benzol und aliphatischen organischen Verbindungen wird ausgeschleust, bevorzugt aus dem Tank, in dem das "Rückbenzol" (d.2) bis zu seiner Verwendung zwischengelagert wird, um die Gesamtmenge der in den Prozess zurückgeführten aliphatischen organischen Verbindungen zu verringern. Die Ausschleusung kann dabei kontinuierlich oder diskontinuierlich vorgenommen werden, bevorzugt aus dem Tank, in dem das "Rückbenzol" (d.2) bis zu seiner Verwendung zwischengelagert wird oder aus dessen zu- oder abführenden Leitungen. Die Menge des auszuschleusenden Gemisches aus Benzol und aliphatischen organischen Verbindungen wird so gewählt, dass die erfindungsgemäß geforderte Spezifikation des Einsatzbenzols (a.1) im Hinblick auf aliphatische organische Verbindungen eingehalten wird. Die Menge des auszuschleusenden Gemisches aus Benzol und aliphatischen organischen Verbindungen ist daher vom Gehalt des frischen zugeführten Benzols an aliphatischen organischen Verbindungen abhängig. Die Hauptmenge des Gemisches aus Benzol und aliphatischen organischen Verbindungen wird in die Nitrierung zurückgeführt. Das ausgeschleuste, nicht in die Nitrierung zurückgeführte organische Material kann verbrannt oder zur Rückgewinnung von Benzol destillativ aufgearbeitet werden oder der petrochemischen Industrie zur Verfügung gestellt werden. Destillativ zurückgewonnenes Benzol wird bevorzugt dem Strom (a.1) zugesclalagen.

Die Abtrennung der Leichtsieder kann durch Einsatz einer geeigneten, selektiv-permeablen Membran unterstützt werden.

### Zweite Variante:

In der zweiten Variante des erfindungsgemäßen Verfahrens erfolgt die Nitrierung in zwei hintereinander geschalteten Nitrieranlagen, wobei in der zweiten Nitrieranlage (Nebenanlage, 200) als Einsatzbenzol überwiegend bis vollständig das in der ersten Nitrieranlage (Hauptanlage, 100) anfallende "Rückbenzol" eingesetzt wird.

Die erste Nitrieranlage (100) umfasst bevorzugt mehrere parallel laufende Reaktionsstraßen, besonders bevorzugt zwei bis vier parallel laufende Reaktionsstraßen. Die zweite Nitrieranlage (200) umfasst bevorzugt nur eine Reaktionsstraße. Die erste Nitrieranlage wird kontinuierlich betrieben. Die zweite Nitrieranlage kann, muss jedoch nicht, ebenfalls kontinuierlich betrieben werden. Aufgrund des geringeren Durchsatzes (das Einsatzbenzol der zweiten Nitrieranlage besteht überwiegend bis vollständig aus dem "Rückbenzol" der ersten Reaktionsstraße, also nur dem überstöchiometrisch eingesetzten Benzol) kann die zweite Nitrieranlage auch diskontinuierlich betrieben werden. Im Falle einer Produktionsunterbrechung in der zweiten Nitrieranlage während des Betriebs der ersten Nitrieranlage wird das anfallende Rückbenzol in einem Puffertank zwischengelagert.

In der ersten Nitrieranlage besteht das Einsatzbenzol (zur Unterscheidung vom Einsatzbenzol der zweiten Nitrieranlage im Folgenden mit (a.1.1) bezeichnet; für die anderen Verfahrensströme wird analog verfahren) aus Benzol, das einen Gehalt an aliphatischen organischen Verbindungen von kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse von (a.1.1), aufweist, . Bevorzugt handelt es sich hierbei um solches Benzol, das nicht zuvor bereits als Edukt einer Nitrierung diente (sog. "Frischbenzol"). Auf die zusätzliche Verwendung von "Rückbenzol" wird hier bewusst verzichtet. Da kommerziell erhältliches Frischbenzol in der Regel einen Gehalt an aliphatischen organischen Verbindungen aufweist, der den erfindungsgemäßen Anforderungen genügt, kann dieses meist direkt eingesetzt werden. Sollte jedoch nur solches Frischbenzol zur Verfügung stehen, dessen Reinheit den erfindungsgemäßen Anforderungen nicht genügt, so muss dieses vor seinem Einsatz in Schritt a) der ersten Nitrieranlage gereinigt werden, bevorzugt durch Destillation. Aufgrund des geringen Gehalts an aliphatischen organischen Verbindungen im Einsatzbenzol (a.1.1) und dem bewussten Verzicht auf die Verwendung von rezykliertem Benzol kann die erste Nitrieranlage ohne Probleme betrieben werden, und zwar selbst dann, wenn in der ersten Nitrieranlage auf eine gezielte Ausschleusung von aliphatischen organischen Verbindungen verzichtet wird, was bevorzugt ist. Wenn jedoch auch in der ersten Nitrieranlage aliphatische organische Verbindungen gezielt ausgeschleust werden sollen, kann dies bevorzugt wie oben für die erste Variante geschildert über die Ausschleusung eines Leichtsiederstroms aus der Gasphase des in Schritt b) eingesetzten Phasentrennapparats geschehen. Die erste Nitrieranlage kann, mit Ausnahme der Besonderheit des Verzichts auf die Verwendung von Rückbenzol als Bestandteil des Einsatzbenzols, nach einem beliebigen aus dem Stand der Technik bekannten adiabaten Nitrierverfahren betrieben werden. Das in Schritt d) der ersten Nitrieranlage erhaltene "Rückbenzol" (d.2.1) wird nicht in Schritt a) *der ersten Nitrieranlage* rezykliert, sondern, ggf. vermischt mit anderem Benzol, bevorzugt Frischbenzol, als Einsatzbenzol (a.1.2) *der zweiten Nitrieranlage* verwendet. Die Einhaltung der oben definierten erfindungsgemäßen Anforderungen an den Gehalt an aliphatischen organischen Verbindungen im Einsatzbenzol (kleiner als 1,5 Massen-%, bevorzugt von kleiner als 0,50 Massen-%, besonders bevorzugt von kleiner als 0,20 Massen-%, ganz besonders bevorzugt von kleiner als 0,10 Massen-%, jeweils bezogen auf die Gesamtmasse des Einsatzbenzols) ist für das Einsatzbenzol der zweiten Nitrieranlage (a.1.2) nicht zwingend erforderlich. Mit Ausnahme dieser Besonderheit kann die zweite Nitrieranlage nach einem beliebigen aus dem Stand der Technik bekannten adiabaten Nitrierverfahren betrieben werden. Das in der zweiten Nitrieranlage nach Abtrennung der wässrigen, Schwefelsäure umfassenden Phase (b.1.2) in einem Phasentrennapparat erhaltene Roh-Nitrobenzol (b.2.2) wird bevorzugt mit dem Roh-Nitrobenzol der ersten Nitrieranlage (b.2.1) vereint und gemeinsam weiter in den Schritten c) und d) aufgearbeitet. In dieser bevorzugten Ausgestaltung der Variante mit zwei Nitrieranlagen gibt es also nur einen "Rückbenzolstrom" (d.2) aus Schritt d), welcher aus der Destillation der vereinigten Roh-Nitrobenzolströme (b.2.1) und (b.2.2) stammt. Die gezielte Ausschleusung der aliphatischen organischen Verbindungen aus der ersten Nitrieranlage (100) erfolgt in dieser Ausführungsform - mit Ausnahme einer ungezielten Ausschleusung, die über das Abgas immer bis zu einem gewissen Grad erfolgt und die daher auch in der ersten Nitrieranlage vorkommt - über die Ausschleusung (anstelle der sonst üblichen *Rückführung*) des "Rückbenzols" (d.2.1). Zur Ausgestaltung der Ausschleusung von aliphatischen organischen Verbindungen aus der zweiten Nitrieranlage stehen verschiedene Möglichkeiten zur Verfügung:

Das Roh-Nitrobenzol aus der zweiten Nitrieranlage (b.2.2) kann vor der Vereinigung mit dem Roh-Nitrobenzol der ersten Nitrieranlage (b.2.1) in einer Destillationskolonne von aliphatischen organischen Verbindungen und überschüssigem Benzol befreit werden. Eine solche Destillationskolonne wird aufgrund der Möglichkeit des Vorhandenseins von Säureresten im Strom (b.2.2) bevorzugt korrosionsbeständig ausgelegt. Der hierbei erhaltene Leichtsiederstrom aus aliphatischen organischen Verbindungen und überschüssigem Benzol kann auf verschiedene Arten weiter behandelt werden. Bei geringem Benzolgehalt wird der Leichtsiederstrom bevorzugt verbrannt. Ist der Benzolgehalt des Leichtsiederstroms hinreichend hoch, kann jedoch eine Wiedergewinnung oder Nutzbarmachung des darin enthaltenen Benzols wirtschaftlich sinnvoller sein. Ein Gegenstand der vorliegenden Erfindung ist daher auch ein Verfahren, bei dem ausgeschleuste aliphatische organische Verbindungen teilweise bis vollständig zur Rückgewinnung von mit den aliphatischen organischen Verbindungen gemeinsam ausgeschleustem Benzol destillativ aufgearbeitet werden. Bevorzugt werden die aliphatischen organischen Verbindungen abdestilliert, wobei Benzol als Sumpfstrom der Destillation gewonnen wird. Das zurückgewonnene Benzol wird bevorzugt dem Strom (a.1.1) zugeschlagen. Diese Vorgehensweise ist dann bevorzugt, wenn der Benzol-Gehalt im Leichtsiederstrom größer als 50,0 Massen-%, bevorzugt größer als 70,0 Massen-%, jeweils bezogen auf die Gesamtmasse des Leichtsiederstroms, ist. Die abgetrennten aliphatischen organischen Verbindungen werden bevorzugt verbrannt. Ganz besonders bevorzugt ist die Rückgabe dieses Leichtsiederstromes an den Lieferanten des Frischbenzols. Daneben besteht die Möglichkeit, da die zweite Nitrieranlage wesentlich unempfindlicher ist gegen erhöhte Gehalte an aliphatischen organischen Verbindungen, den Leichtsiederstrom direkt, also ohne destillative Auftrennung in aliphatische organische Verbindungen und Benzol, zumindest teilweise als zusätzlichen Bestandteil des Einsatzbenzols (a.1.2) einzusetzen.

Die oben für die erste Variante geschilderten Möglichkeiten der gezielten Ausschleusung aliphatischer organischer Verbindungen können grundsätzlich auch in der Nitrieranlage (200) der zweiten Variante eingesetzt werden. Dies ist jedoch nicht zwingend erforderlich, da ggf. erhöhte Gehalte an aliphatischen organischen Verbindungen in der kleinen Nebenanlage (200) weit weniger stark stören als in der Hauptanlage (100).

Eine mögliche Ausführungsform der zweiten Variante wird im Folgenden mit Hilfe der beigefügten Zeichnung Fig. 2 näher erläutert:

### Erste Nitrieranlage (Hauptanlage)

Einem Reaktor (1) werden ein Schwefelsäure- (11) = (a.2.1), ein Salpetersäure- (12) = (a.3.1) und ein Frischbenzolstrom (13) = (a.1.1) zugeführt. Nach vollständiger Umsetzung der Salpetersäure mit dem Benzol zu Nitrobenzol unter adiabater Reaktionsführung wird das nunmehr ca. 130 °C heiße Reaktionsprodukt (14) einem Phasentrennapparat (2) zugeführt, in dem das Reaktionsprodukt (14) in eine organische Phase ((15) = (b.2.1)= Roh-Nitrobenzol, enthaltend neben Nitrobenzol noch Benzol und Leichtsieder) und eine wässrige Phase ((16) = (b.1.1) = Absäure, enthaltend neben Schwefelsäure noch geringe Anteile an Nitrobenzol und Benzol) zerfällt. Die wässrige, hauptsächlich Schwefelsäure umfassende Phase (16) wird im Verdampfer (3) durch plötzliche Druckerniedrigung einer Blitzverdampfung von Wasser unterzogen und auf diese Weise aufkonzentriert. Die aufkonzentrierte Schwefelssäure (17) wird im Schwefelsäuretank (4) zur erneuten Verwendung gelagert. Nach dem Abtrennen im Phasentrennapparat wird das Rohnitrobenzol (15) = (b.2.1) in der Rohnitrobenzolkühlung (5) auf ca. 50 °C abgekühlt und der Wäsche (6) zugeführt. Der so erhaltene von Nitrophenolen und Salzen weitgehend befreite Strom des vorgereinigten Nitrobenzols (18) wird wieder aufgeheizt und in einer Destillationskolonne (7) von Wasser, Benzol und noch vorhandenen Leichtsiedern, welche über Kopf abgetrennt werden (19), befreit, wodurch Rein-Nitrobenzol (20) = (d.1.1) erhalten und in Tank (8) gelagert wird. Das kondensierte Kopfprodukt (19) der Destillationskolonne (7) wird einem Phasentrennapparat (9) zugeführt, in dem das Kopfprodukt in eine organische Phase ((21) = (d.2.1), enthaltend Benzol) und eine wässrige Phase (22) zerfällt. Die organische Phase (21) = (d.2.1) wird in Tank (10) gefahren.

### Zweite Nitrieranlage (kleiner, einstraßige Nebenanlage)

Einem Reaktor (1*) werden ein Schwefelsäure- (11*) = (a.2.2), ein Salpetersäure- (12*) = (a.3.2), optional ein Frischbenzolstrom (13*) und die organische Phase (21)= (d.2.1) aus Tank (10) zugeführt. Nach vollständiger Umsetzung der Salpetersäure mit dem Benzol zu Nitrobenzol unter adiabater Reaktionsführung wird das nunmehr ca. 130 °C heiße Reaktionsprodukt (14*) einem Phasentrennapparat (2*) zugeführt, in dem das Reaktionsprodukt (14*) in eine organische Phase ((15*) = (b.2.2) = Roh-Nitrobenzol, enthaltend neben Nitrobenzol noch Benzol und Leichtsieder) und eine wässrige Phase ((16*) =(b.1.2) = Absäure, enthaltend neben Schwefelsäure noch geringe Anteile an Nitrobenzol und Benzol) zerfällt. Die wässrige, hauptsächlich Schwefelsäure umfassende Phase (16*) = (b.1.2) wird mit der hauptsächlich Schwefelsäure umfassende Phase (16) = (b.1.1) der vor gelagerten Frischbenzol-Nitrierung in der ersten Nitrieranlage vereinigt und gemeinsam im Verdampfer (3) durch plötzliche Druckerniedrigung einer Blitzverdampfung von Wasser unterzogen und auf diese Weise ebenfalls aufkonzentriert. Die so aufkonzentrierte Schwefelsäure (17) wird im Schwefelsäuretank (4) zur erneuten Verwendung gelagert. Nach dem Abtrennen im Phasentrennapparat (2*) wird das Rohnitrobenzols (15*)= (b.2.2) in einer Destillationskolonne (7*) von Leichtsiedern und anteilig Benzol befreit. Dies kann durch direkte oder indirekte Beheizung mit Dampf geschehen. Das an aliphatischen organischen Verbindungen arme Sumpfprodukt (24) = (b.2.2) enthaltend Nitrobenzol, das nicht über Kopf der Destillationskolonne (7*) abgezogen wurde, wird mit dem Rohnitrobenzolstrom (15) = (b.2.1) vereinigt und gemeinsam in der Rohnitrobenzolkühlung (5) auf ca. 50 °C abgekühlt und der Wäsche (6) zugeführt. In dieser Variante gibt es also keinen Rückbenzolstrom *im klassischen Sinne*. Es gibt jedoch einen Rückbenzolstrom (d.2) = (21) aus Schritt d), welcher aus der Destillation der vereinigten Roh-Nitrobenzolströme (b.2.1) = (15) und (b.2.2) = (24) stammt und in der zweiten Nitrieranlage verarbeitet wird. Das Kopfprodukt (23) (der Leichtsiederstrom) der Destillationskolonne (7*) wird im Puffertank (25) zwischengelagert.

In allen Ausführungsformen können zusätzlich auch Teilmengen der aliphatischen organischen Verbindungen über das Abgas der anderen Betriebseinheiten wie Wäschen, Destillation, etc. einer Nitrieranlage ausgeschleust werden.

In allen Ausführungsformen des erfindungsgemäßen Verfahrens ist es bevorzugt, die gezielte Ausschleusung der aliphatischen organischen Verbindungen so auszugestalten, dass der Gehalt an aliphatischen organischen Verbindungen im Rückbenzol (d.2) von 0,1 Massen-% bis 60,0 Massen-%, besonders bevorzugt von 0,1 Massen-% bis 40,0 Massen-%, ganz besonders bevorzugt von 0,1 Massen-% bis 20,0 Massen-%, außerordentlich ganz besonders bevorzugt von 0,0001 Massen-% bis 10,0 Massen-%, jeweils bezogen auf die Gesamtmasse von (d.2), beträgt.

Nachstehend werden die Schritte der Erfindung, die für beide Varianten und alle oben genannten Ausführungsformen der gezielten Ausschleusung der aliphatischen organischen Verbindungen gleich sind, detailliert erläutert. Verschiedene Ausführungsformen sind dabei beliebig miteinander kombinierbar, solange sich für den Fachmann aus dem Kontext nicht eindeutig das Gegenteil ergibt.

**Schritt a)** kann grundsätzlich nach allen aus dem Stand der Technik bekannten adiabaten Nitrierverfahren durchgeführt werden, solange mit diesen die spezifizierten Randbedingungen bzgl. des Benzolüberschusses und der Reinheit der Einsatzstoffe eingehalten werden können. Für die Ausführung dieses Schritts des erfindungsgemäßen Verfahrens wird bevorzugt ein Rohrreaktor eingesetzt, in dem mehrere Dispergierelemente über die Länge des Reaktors verteilt angeordnet sind, die eine intensive Dispergierung und Durchmischung von Benzol, Salpetersäure und Schwefelsäure gewährleisten. Ein solcher Reaktor, sowie die Form einsetzbarer Dispergierelemente, sind beispielsweise in EP 0708 076 B1 (Figur 2) und EP 1 291 078 A2 (Figur 1) beschrieben. Bevorzugt wird Schritt a) in einer Verfahrensführung ausgeführt, wie sie in DE 10 2008 048 713 A1, insbesondere Absatz [0024], beschrieben ist.

Die Phasentrennung in **Schritt b)** erfolgt ebenfalls nach aus dem Stand der Technik an sich bekannten Verfahren in einem dem Fachmann bekannten Trennbehälter. Die wässrige Phase (b.1) enthält im Wesentlichen (infolge der Bildung von Reaktionswasser und durch das Einschleppen von Wasser in die Reaktion aus der eingesetzten Salpetersäure verdünnte) Schwefelsäure neben anorganischen Verunreinigungen, die organische Phase (b.2) enthält im Wesentlichen Nitrobenzol neben überschüssigem Benzol und organischen Verunreinigungen.

Die Aufkonzentrierung der wässrigen Phase (b.1) in **Schritt c)** erfolgt grundsätzlich wie aus dem Stand der Technik bekannt. Die Schwefelsäure in der wässrigen Phase wird in einem Entspannungsverdampfer (auch als Blitzverdampfer bezeichnet) aufkonzentriert, indem Wasser in einen Bereich reduzierten Druckes hinein verdampft wird. Bei richtiger Wahl der Reaktionsbedingungen in der adiabaten Nitrierung von Benzol mit Mischsäure erzielt man mit der Reaktionswärme der exothermen Reaktion eine so starke Erhitzung der Schwefelsäure enthaltenden wässrigen Phase (b.1), dass im Entspannungsverdampfer gleichzeitig wieder die Konzentration und Temperatur der Schwefelsäure enthaltenden wässrigen Phase eingestellt werden kann, die diese vor der Reaktion mit Benzol und Salpetersäure bei Eintritt in den Reaktorraum hatte, d. h., (c.1) entspricht hinsichtlich Temperatur und Konzentration (a.2). Dies ist beschrieben in EP 2 354 117 A1, insbesondere Absatz [0045].

Die Aufarbeitung der organischen Phase (b.2) in **Schritt d)** erfolgt grundsätzlich wie aus dem Stand der Technik bekannt. Eine bevorzugte Verfahrensweise wird nachstehend geschildert:
Die organische Phase (b.2), die üblicherweise noch Spuren an Säure enthält, wird in einer bis zwei Wäschen, bevorzugt einer Wäsche, mit einer wässrigen Waschflüssigkeit gewaschen und dann von der sauren wässrigen Phase durch Phasentrennung getrennt (bei mehreren Wäschen nach jeder einzelnen Wäsche). Bei diesem Vorgang werden die Säurereste, die das rohe Nitrobenzol (b.2) enthält, ausgewaschen; daher wird dieser Verfahrensschritt auch als *saure Wäsche* bezeichnet. Dieser Schritt ist aus dem Stand der Technik hinlänglich bekannt und wird daher hier nur kurz umrissen. Bevorzugt werden zur Durchführung dieser sauren Wäsche im Betrieb anfallende wässrige Ströme rezyklisiert. **(Schritt d(i).)**

Die so erhaltene organische Phase wird anschließend in einer bis zwei, bevorzugt einer alkalischen Wäsche(n) mit einer wässrigen Lösung einer Base, bevorzugt ausgewählt aus Natriumhydroxid, Natriumcarbonat oder Natriumhydrogencarbonat, gewaschen und anschließend von dem alkalischen Waschwasser durch Phasentrennung getrennt (bei mehreren Wäschen nach jeder einzelnen Wäsche). Besonders bevorzugt wird als wässrige Baselösung Natronlauge eingesetzt. Dieser Schritt ist aus dem Stand der Technik hinlänglich bekannt und wird daher hier nur kurz umrissen. Der pH-Wert der eingesetzten Natronlauge und ihr Massenverhältnis zur organischen Phase werden so eingestellt, dass acide Verunreinigungen (z. B. als Nebenprodukte gebildete Nitrophenole und in Schritt d(i)) nicht vollständig entfernte Säurereste) in dieser/diesen alkalischen Wäsche(n) weitgehend bis vollständig, bevorzugt vollständig, neutralisiert werden. Die anschließende Aufarbeitung des alkalischen Abwassers kann nach den Verfahren des Standes der Technik, z. B. gemäß EP 1 593 654 A1 und EP 1 132 347 A2 erfolgen. **(Schritt d(ii)**.)

Die so erhaltene organische Phase wird schließlich in mindestens einer, bevorzugt zwei bis vier, besonders bevorzugt zwei bis drei, ganz besonders bevorzugt zwei, neutralen Wäsche(n) mit Wasser gewaschen und anschließend von der wässrigen Phase durch Phasentrennung getrennt (bei mehreren Wäschen nach jeder einzelnen Wäsche). Dies kann grundsätzlich nach allen im Stand der Technik üblichen Verfahren geschehen. Als Waschwasser wird dabei bevorzugt vollentsalztes Wasser (VE-Wasser), besonders bevorzugt eine Mischung aus VE-Wasser und Dampfkondensat (d. i. ein Kondensat von Wasserdampf, welcher durch Wärmeaustausch von Wasser mit beliebigen exothermen Verfahrensschritten erhalten wurde) und ganz besonders bevorzugt Dampfkondensat eingesetzt.

Bevorzugt ist eine Verfahrensweise, bei der in der letzten neutralen Wäsche eine Elektrophorese eingesetzt wird (siehe WO 2012/013678 A2). **(Schritt d(iii).**)

Das gewaschene Nitrobenzol wird schließlich von gelöstem Wasser, nicht umgesetztem Benzol und ggf. organischen Verunreinigungen durch weitere Aufarbeitung befreit. Diese Aufarbeitung erfolgt bevorzugt durch Destillation, wobei über Kopf die Brüden Wasser und Benzol und ggf. organische Verunreinigungen ausgetrieben werden. Die Brüden werden gekühlt und in einen Trennbehälter gefahren. In der unteren Phase setzt sich Wasser ab, das abgetrennt wird. In der oberen Phase befinden sich Benzol und Leichtsieder, die als Rückbenzol (d.2) wieder der Reaktion zugeführt werden. Bevorzugt wird als Destillationsapparat eine Rektifizierkolonne eingesetzt. Das Sumpfprodukt der Destillation wird, ggf. nach einer weiteren Destillation, in welcher Nitrobenzol *als Destillat* (also als Kopf- oder Seitenstromprodukt) erhalten wird, als Rein-Nitrobenzol (d.1) weiteren Anwendungen (wie der Hydrierung zu Anilin) zugeführt. **(Schritt d(iv).)**

Wird der Gehalt an aliphatischen organischen Verbindungen im Einsatzbenzol (a.1 bzw. a.1.1) minimiert, so ergeben sich die folgenden Vorteile:
i) Das im Trennbehälter von Schritt b) anfallende an aliphatischen organischen Verbindungen arme Rohnitrobenzol bedarf weniger Kühlmedium zum Abkühlen vor dem Eintritt in die saure Wäsche.
ii) Die Auflieizung des an aliphatischen organischen Verbindungen armen Rohnitrobenzols Rohnitrobenzols, wie sie bei der Destillation nach der Wäsche erforderlich ist, benötigt weniger Heizmedium.
iii) Die Bildung von Nebenprodukten in der Reaktion wie Pikrinsäure wird minimiert.
iv) Die Schwefelsäureverluste in der sauren Wäsche fallen geringer aus.
v) Die aliphatenfreie Nitrierung von Benzol hat außerdem den Vorteil, dass die hydraulische Belastung in der Reaktion geringer ausfällt und somit eine höhere Produktionskapazität zur Verfügung steht.
vi) Phasentrennprobleme in Schritt d(iii) werden durch verkürzte Phasentrennzeiten minimiert, wodurch der Investaufvvand für einen Apparat geringer ist.
vii) Die Bildung von Nebenprodukten in der Reaktion wie Stickoxide (NOx) wird minimiert.

Bei der Herstellung von Nitrobenzol durch adiabate Nitrierung von Benzol mit einer kontinuierlichen oder diskontinuierlichen Austragung von aliphatischen organischen Verbindungen aus dem Prozesskreislauf werden eine höhere Anlagenproduktivität, geringere Energiekosten und eine bessere Produktqualität erreicht, wenn die Nitrierung mit Einsatzbenzol, das der erfindungsgemäßen Spezifikation genügt, durchgeführt wird.

### Beispiele

Gehaltsangaben in ppm oder % sind, sofern nicht anders angegeben, Massenanteile bezogen auf die Gesamtmasse des jeweiligen Stoff(strom)s. Analysenwerte wurden mittels Gaschromatographie bestimmt.

### Beispiele 1 bis 5

Die Nitrierung wurde mit einem Verfahren wie in Fig. 1 gezeigt durchgeführt. Fig. 1 zeigt ein Verfahren, bei dem nur die erste Nitrieranlage (Hauptanlage) aus Fig. 2 eingesetzt wird. Die Bezugszeichen der Fig. 1 haben die gleiche Bedeutung wie weiter oben für die Hauptanlage von Fig. 2 erläutert. Das Frischbenzol enthielt organische aliphatische Verbindungen (nachfolgend Aliphaten) im Bereich von 200 ppm bis 2000 ppm. Der auf Salpetersäure bezogene stöchiometrische Benzolüberschuss wurde auf Werte von 4,0 % bis 10 % der Theorie eingestellt. Das Rückbenzol (21) = (d.2) enthielt von 10 Massen-% bis 50 Massen% Aliphaten, bezogen auf die Gesamtmasse des Rückbenzols. Aus dem jeweiligen Aliphatengehalt im Frischbenzol und dem entsprechenden Aliphatengehalt im Rückbenzol ergibt sich der resultierende Aliphatengehalt im Einsatzbenzol. Es zeigt sich, dass mit steigendem Aliphatengehalt im Einsatzbenzol der Schwefelsäureverlust ansteigt und sich die Pikrinsäurewerte erhöhen. Die Aliphaten wurden im Phasentrennapparat bei einem Stickstoffüberdruck von 53 mbar ausgeschleust. Die Temperatur der Nitrierlösung betrug 130 °C bis 133 °C. Die Kondensation des Abgases des Phasentrennapparates erfolgte einstufig.

### Beispiel 6

Die Nitrierung wurde in einer ersten Nitrieranlage (der Hauptanlage) mit dahinter geschalteter zweiter Nitrieranlage (der Nebenanlage) mit einem Verfahren wie in Fig. 2 gezeigt durchgeführt.

Die Hauptanlage wurde ausschließlich mit Frischbenzol (13), das organische aliphatische Verbindungen (nachfolgend Aliphaten) von 1013 ppm enthielt, betrieben. Das dabei entstehende Rückbenzol (21) = (d.2.1) enthielt ca. 1,818 Massen-% Aliphaten, bezogen auf die Gesamtmasse des Rückbenzols und wurde in die Reaktion (1*) der Nebenanlage gefahren. Ferner wurden in dem Reaktor (1*) der Nebenanlage noch ein Schwefelsäure- (11*) = (a.2.2) und ein Salpetersäurestrom (12*) = (a.3.2) zugeführt. Der auf Salpetersäure bezogene stöchiometrische Benzolüberschuss wurde in Haupt- und Nebenanlage auf Werte von 4,0 % bis 10 % der Theorie eingestellt. Es zeigt sich, dass in der Hauptanlage mit dem sehr geringen Aliphatengehalt von ca. 1000 ppm im Einsatzbenzol nur sehr geringe Schwefelsäureverluste vorliegen und die Pikrinsäurewerte im Roh-Nitrobenzol sehr niedrig sind. In der Nebenanlage wurde im Phasentrennapparat (2*) die Kreislaufschwefelsäure als untere Phase abgetrennt (16*) und mit der Kreislaufschwefelsäure aus der Hauptanlage (Strom 16) vereinigt und im Verdampfer (3) der Hauptanlage aufkonzentriert. Die obere organische Phase im Trennapparat wurde in die Destillationskolonne (7*) der Nebenanlage gefahren, in der das Nitrobenzol von Benzol, Aliphaten und Spuren von Wasser befreit wurde und als Strom (24) in die Hauptanlage zurückgeführt wurde und dort mit dem Rohnitrobenzol (Strom 15) vereinigt wurde und im Wärmetauscher (5) der Hauptanlage abgekühlt wurde. Die Brüden der Destillationskolonne wurden in einem Phasentrennapparat von Wasser befreit und die organische Phase (23) wurde in einen Puffertank (25) gefahren.

Nachfolgende Tabelle 1 beschreibt die Beispiele 1 bis 6.

**Tabelle 1**

| Beispiel | Benzolüberschuss [% der Theorie bezogen auf Salpetersäure] ^{[a]} | Aliphaten-Gehalt im Frischbenzol^{[a]} [ppm] | Aliphaten-Gehalt im Rückbenzol, das Reaktor 1 zugeführt wird [%] | Aliphaten-Gehalt im Einsatzbenzol von Reaktor 1^{[b]} [%] | Schwefelsäureverlust über Strom 15^{[c]} [%] | PikrinsäureGehalt in Strom 15 [ppm] |
|---|---|---|---|---|---|---|
| 1 (Vergleich) | 5,565 | 788 | 48 | 4,43527 | 1,7 | 431 |
| 2 (Vergleich) | 4,880 | 894 | 41 | 3,11981 | 1,5 | 357 |
| 3 (Vergleich) | 5,612 | 993 | 31 | 2,38824 | 1,0 | 228 |
| 4 (erfindungsgemäß) | 6,502 | 975 | 19 | 1,49768 | 0,5 | 95 |
| 5 (erfindungsgemäß) | 5,231 | 953 | 12 | 0,76915 | 0,4 | 113 |
| 6 (erfindungsgemäß), mit Haupt- und Nebenanlage (ohne Rückbenzol in der Hauptanlage) | 5,899 | 1013 | --- (kein Rückbenzolstrom in Reaktor 1 (Hauptanlage)) | 0,1013 | 0,3 | 87 |

| | | | | | | |
|---|---|---|---|---|---|---|
| [a] In Beispiel 6 gilt die Angabe jeweils für Haupt- und Nebenanlage. [b] Der Aliphatengehalt im Einsatzbenzols des Reaktors 1* (Beispiel 6, Nebenanlage) betrug 1,818 Massen-%. [c] Bei zu hohem Gehalt an Aliphaten in Strom 14 kann es infolge deren geringen Siedepunktes zum "Sprudeln" im Phasentrennapparat 2 kommen. Hierdurch gelangt Schwefelsäure in die organische Phase Strom 15 und geht über die Wäsche verloren, anstatt über Strom 16 rezykliert zu werden. | | | | | | |

Die Beispiele zeigen, dass mit dem erfindungsgemäßen Verfahren die Schwefelsäureverluste minimiert und der Pikrinsäuregehalt des Roh-Nitrobenzols deutlich reduziert werden können.

### Beispiel 7: illustration des Zusammenhanges zwischen Phasentrennung und Aliphatengehalt

Zur Untersuchung des Einflusses des Aliphatengehalts auf die Phasentrennung einer Mischung aus Nitrobenzol, ggf. Benzol und Aliphaten wurden Nitrobenzol und Nitrobenzol-Benzol-Gemische mit Aliphaten bis zu einem Gehalt von 3,89 Massen-%, jeweils bezogen auf die Gesamtmasse der organischen Mischung, versetzt. Der Aliphatengehalt des verwendeten Nitrobenzols war vernachlässigbar gering (< 100ppm) und wurde daher bei den folgenden Betrachtungen nicht berücksichtigt. Das Nitrobenzol war frei von Nitrophenolen und Säure. Damit wurde also der Fall einer neutralen Wäsche von Rohnitrobenzol (Schritt d(iii)) nachgestellt. Durch die Variation des Aliphatengehaltes wurde geprüft, ob sich eine vorherige Aliphatenabtrennung in der Wäsche bemerkbar macht.

Beispiel 7a: In einem beheizbaren Scheidetrichter wurden Nitrobenzol und vollentsalztes Wasser (VE-Wasser; Massenverhältnis organische Phase zu VE-Wasser = 2,8) 10 min bei 40 °C und 600 U/min mit einem Scheibenrührer gerührt. Anschließend wurde der Rührer abgestellt und die Zeit bis zur vollständigen Phasentrennung ermittelt.

Beispiel 7b: In einem beheizbaren Scheidetrichter wurden Nitrobenzol und Benzol (8,74 % der Masse des Nitrobenzols) und VE-Wasser (Verhältnis organische Phase zu VE-Wasser = 2,8) 10 min bei 40 °C und 600 U/min mit einem Scheibenrührer gerührt. Anschließend wurde der Rührer abgestellt und die Zeit bis zur vollständigen Phasentrennung ermittelt.

Beispiele 7c und 7d: In einem beheizbaren Scheidetrichter wurden Nitrobenzol und ein Benzol-/Aliphatengemisch und VE-Wasser (Verhältnis organische Phase zu VE-Wasser = 2,8) 10 min bei 40 °C und 600 U/min mit einem Scheibenrührer gerührt. Anschließend wurde der Rührer abgestellt und die Zeit bis zur vollständigen Phasentrennung ermittelt.

Die Ergebnisse in Tabelle 2 zeigen, dass sich ein niedriger Aliphatengehalt in der neutralen Wäsche positiv auf die Phasentrennzeit auswirkt und damit bereits durch eine teilweise Abtrennung der Aliphaten eine Verringerung der für die Phasentrennung erforderlichen Beloältergröße bewirkt werden kann.

**Tabelle 2: Phasentrennzeiten in der Wäsche von Nitrobenzol-/Benzol-/Aliphaten-Gemischen mit VE-Wasser**

| Beispiel | Nitrobenzol | Benzol | Zugesetzte Aliphaten | | Aliphatengehalt im Rückbenzol | Phasentrennzeit |
|---|---|---|---|---|---|---|
| | [g] | [g] | [g] | [%] | [%]^{[a]} | [sec] |
| 7a | 299,98 | 0 | 0 | 0 | - | 16 |
| 7b | 299,97 | 26,22 | 0 | 0 | 0 | 18 |
| 7c | 299,95 | 24,82 | 1,16 | 0,36 | 4,46 | 21 |
| 7d | 299,99 | 20,99 | 4,92 | 1,51 | 18,99 | 27 |
| 7e | 299,98 | 20,88 | 12,47 | 3,72 | 37,39 | 42 |

| | | | | | | |
|---|---|---|---|---|---|---|
| [a] Berechnet für den Fall, dass das in der Mischung aus Nitrobenzol, ggf. Benzol und Aliphaten enthaltene Benzol vollständig einer Nitrierung zugeführt wird. | | | | | | |

Die Ergebnisse zeigen, dass die Phasentrennzeit mit dem Aliphatengehalt ansteigt.

## Patentansprüche

1. Kontinuierlich betriebenes adiabates Verfahren zur Herstellung von Nitrobenzol durch Nitrierung von Benzol in einer Nitrieranlage, bei dem
a) ein Benzol-haltiger Strom (a.1), der mindestens 90 Massen-%, Benzol, bezogen auf die Gesamtmasse von (a.1), umfasst, in einem Reaktor mit einem Gemisch aus Schwefelsäure (a.2) und Salpetersäure (a.3) unter adiabaten Bedingungen umgesetzt wird, wobei Benzol in einem stöchiometrischen Überschuss bezogen auf Salpetersäure (a.3) eingesetzt wird,
b) das in Schritt a) erhaltene Verfahrensprodukt in einem Phasentrennapparat in eine wässrige, Schwefelsäure umfassende Phase (b.1) und eine organische, Nitrobenzol umfassende Phase (b.2) getrennt wird,
c) die in Schritt b) erhaltene wässrige Phase (b.1) durch Verdampfung von Wasser zu einer wässrigen Phase (c.1) mit gegenüber (b.1) erhöhter Schwefelsäurekonzentration aufkonzentriert wird, und wobei die Phase (c.1) teilweise bis vollständig in Schritt a) zurückgeführt und als Bestandteil von (a.2) eingesetzt wird,
d) die in Schritt b) erhaltene organische Phase (b.2) zu Rein-Nitrobenzol (d.1) aufgearbeitet wird, wobei ein Benzol-haltiger Strom (d.2) gewonnen wird, der teilweise bis vollständig als Bestandteil von (a.1) in der Nitrieranlage aus Schritt a) verwendet wird,
**dadurch gekennzeichnet, dass**
aliphatische organische Verbindungen an mindestens einer Stelle gezielt so aus dem Prozess ausgeschleust werden, dass dem Reaktor nur ein solcher Benzol-haltiger Strom (a.1) zugeführt wird, der einen Gehalt an aliphatischen organischen Verbindungen von kleiner als 1,5 Massen-%, bezogen auf die Gesamtmasse von (a.1), aufweist.

2. Adiabates Verfahren zur Herstellung von Nitrobenzol durch Nitrierung von Benzol in zwei hintereinander geschalteten Nitrieranlagen (100, 200) mit mindestens je einem Reaktor (1, 1*), wobei
a) im Reaktor (1) der ersten, kontinuierlich betriebenen, Nitrieranlage (100) ausschließlich Benzol (a.1.1), das einen Gehalt an aliphatischen organischen Verbindungen von kleiner als 1,5 Massen-%, bezogen auf die Gesamtmasse von (a.1.1), aufweist, mit einem Gemisch aus Schwefelsäure (a.2.1) und Salpetersäure (a.3.1) unter adiabaten Bedingungen umgesetzt wird, wobei Benzol in einem stöchiometrischen Überschuss bezogen auf Salpetersäure (a.3.1) eingesetzt wird;
b) das in Schritt a) erhaltene Verfahrensprodukt in einem Phasentrennapparat (2) in eine wässrige, Schwefelsäure umfassende Phase (b.1.1) und eine organische, Nitrobenzol umfassende Phase (b.2.1) getrennt wird,
c) die in Schritt b) erhaltene wässrige Phase (b.1.1) durch Verdampfung von Wasser zu einer wässrigen Phase (c.1.1) mit gegenüber (b.1.1) erhöhter Schwefelsäurekonzentration aufkonzentriert wird, und wobei die Phase (c.1.1) teilweise bis vollständig in Schritt a) zurückgeführt und als Bestandteil von (a.2.1) eingesetzt wird,
d) die in Schritt b) erhaltene organische Phase (b.2.1) zu Rein-Nitrobenzol (d.1.1) * aufgearbeitet wird, wobei ein Benzol-haltiger Strom (d.2.1) gewonnen wird,
und bei dem der Benzol-haltige Strom (d.2.1) in der zweiten, kontinuierlich oder diskontinuierlich betriebenen, Nitrieranlage (200) in einem Reaktor (1*) mit einem Gemisch aus Schwefelsäure (a.2.2) und Salpetersäure (a.3.2) unter adiabaten Bedingungen zu Nitrobenzol umgesetzt wird, wobei Benzol in einem stöchiometrischen Überschuss bezogen auf Salpetersäure (a.3.2) von bevorzugt 2,0 % bis 20 %, besonders bevorzugt von 5,0 % bis 10 % der Theorie, eingesetzt wird, und wobei das so gebildete Nitrobenzol anschließend zu Rein-Nitrobenzol aufgearbeitet wird.

3. Verfahren nach Anspruch 1 oder 2, bei dem die aliphatischen organischen Verbindungen ausgewählt sind aus der Gruppe bestehend aus Cyclohexan, Heptan, Methylcyclohexan, Bicycloheptan, Isomeren des Dimethylcyclopentans, Ethylcyclopentan, Pentan, Cyclopentan und 2-Methylhexan.

4. Verfahren nach einem der Ansprüche 1 bis 3, bei dem der relativ zum Atmosphärendruck in der Gasphase des in Schritt b) eingesetzten Phasentrennapparats gemessene Druck auf einen Wert von 20 mbar bis 200 mbar eingestellt wird, und bei dem die Temperatur in im Schritt b) eingesetzten Phasentrennapparat auf einen Wert einen Wert von 100 °C bis 140 °C, eingestellt wird, und bei dem aliphatische organische Verbindungen mit der Gasphase des in Schritt b) eingesetzten Phasentrennapparats ausgeschleust werden.

5. Verfahren nach Anspruch 4, bei dem zur Unterstützung der Ausschleusung der aliphatischen organischen Verbindungen ein Inertgas in den Phasentrennapparat eingespeist wird, mit dem zusammen aliphatische organische Verbindungen über eine an den Gasraum des Phasentrennapparats angeschlossene Abgasleitung ausgeschleust werden.

6. Verfahren nach einem der Ansprüche 2 bis 5, bei dem das in der adiabaten Umsetzung in Reaktor (1*) erhaltene Verfahrensprodukt in einem Phasentrennapparat (2*) in eine wässrige, Schwefelsäure umfassende Phase (b.1.2) und eine organische, Nitrobenzol umfassende Phase (b.2.2) getrennt wird, und wobei die organische Phase (b.2.2) = (15*) in einer Destillationskolonne (7*) von aliphatischen organischen Verbindungen und überschüssigem Benzol befreit und danach mit der organischen Phase (b.2.1) = (15) der ersten Nitrieranlage (100) vermischt wird.

7. Verfahren nach einem der Ansprüche 1 und 3 bis 6, bei dem ausgeschleuste aliphatische organische Verbindungen teilweise bis vollständig zur Rückgewinnung von mit den aliphatischen organischen Verbindungen gemeinsam ausgeschleustem Benzol destillativ aufgearbeitet werden.

8. Verfahren nach Anspruch 7, bei dem das zurückgewonnene Benzol teilweise bis vollständig als Bestandteil von Strom (a.1) oder (a.1.1) eingesetzt wird.

## Claims

1. Continuous adiabatic process for the preparation of nitrobenzene by the nitration of benzene in a nitration unit, wherein
a) a benzene-containing stream (a.1 1), comprising at least 90 wt% of benzene, based on the total weight of (a.1 1), is reacted in a reactor with a mixture of sulfuric acid (a.2) and nitric acid (a.3) under adiabatic conditions, the benzene being used in a stoichiometric excess, based on nitric acid (a.3),
b) the process product obtained in step a) is separated in a phase separation apparatus into an aqueous phase (b.1) comprising sulfuric acid and an organic phase (b.2) comprising nitrobenzene,
c) the aqueous phase (b.1) obtained in step b) is concentrated by evaporation of the water to give an aqueous phase (c.1) having a higher sulfuric acid concentration than (b.1), and all or part of the phase (c.1) being recycled into step a) and used as a component of (a.2), and
d) the organic phase (b.2) obtained in step b) is worked up to pure nitrobenzene (d.1) to give a benzene-containing stream (d.2), all or part of which is used as a component of (a. 1) in the nitration unit of step a),
**characterized in that**
aliphatic organic compounds are specifically removed from the process at at least one point so that only such a benzene-containing stream (a.1) having a content of aliphatic organic compounds of less than 1.5 wt%, based on the total weight of (a.1 1), is fed into the reactor.

2. Adiabatic process for the preparation of nitrobenzene by the nitration of benzene in a series of two nitration units (100, 200) each having at least one reactor (1, 1*), wherein
a) exclusively benzene (a.1.1) having a content of aliphatic organic compounds of less than 1.5 wt%, based on the total weight of (a. 1.1), is reacted in the reactor (1) of the first, continuously operating nitration unit (100) with a mixture of sulfuric acid (a.2.1) and nitric acid (a.3.1) under adiabatic conditions, the benzene being used in a stoichiometric excess, based on nitric acid (a.3.1),
b) the process product obtained in step a) is separated in a phase separation apparatus (2) into an aqueous phase (b.1.1) comprising sulfuric acid and an organic phase (b.2.1) comprising nitrobenzene,
c) the aqueous phase (b.1.1) obtained in step b) is concentrated by evaporation of the water to give an aqueous phase (c.1.1) having a higher sulfuric acid concentration than (b.1.1), and all or part of the phase (c.1.1) being recycled into step a) and used as a component of (a.2.1), and
d) the organic phase (b.2.1) obtained in step b) is worked up to pure nitrobenzene (d.1.1) to give a benzene-containing stream (d.2.1),
and wherein the benzene-containing stream (d.2.1) is reacted in a reactor (1*) of the second, continuously or discontinuously operating nitration unit (200) with a mixture of sulfuric acid (a.2.2) and nitric acid (a.3.2) under adiabatic conditions to give nitrobenzene, the benzene being used in a stoichiometric excess, based on nitric acid (a.3.2), preferably of 2.0% to 20%, particularly preferably of 5.0% to 10% of theory, and the nitrobenzene formed in this way then being worked up to pure nitrobenzene.

3. Process according to Claim 1 or 2, wherein the aliphatic organic compounds are selected from the group consisting of cyclohexane, heptane, methylcyclohexane, bicycloheptane, isomers of dimethylcyclopentane, ethylcyclopentane, pentane, cyclopentane and 2-methylhexane.

4. Process according to one of Claims 1 to 3, wherein the pressure measured relative to atmospheric pressure in the gas phase of the phase separation apparatus used in step b) is adjusted to a value of 20 mbar to 200 mbar, wherein the temperature in the phase separation apparatus used in step b) is adjusted to a value of 100°C to 140°C, and wherein aliphatic organic compounds are removed with the gas phase of the phase separation apparatus used in step b).

5. Process according to Claim 4, wherein an inert gas is fed into the phase separation apparatus in order to assist the removal of the aliphatic organic compounds, the aliphatic organic compounds being removed together with said inert gas via an off-gas line connected to the gas space of the phase separation apparatus.

6. Process according to one of Claims 2 to 5, wherein the process product obtained in the adiabatic reaction in the reactor (1*) is separated in a phase separation apparatus (2*) into an aqueous phase (b.1.2) comprising sulfuric acid and an organic phase (b.2.2) comprising nitrobenzene, and the organic phase (b.2.2) = (15*) being freed of aliphatic organic compounds and excess benzene in a distillation column (7*) and then mixed with the organic phase (b.2.1) = (15) of the first nitration unit (100).

7. Process according to one of Claims 1 and 3 to 6, wherein all or part of the removed aliphatic organic compounds is worked up by distillation to recover benzene removed together with the aliphatic organic compounds.

8. Process according to Claim 7, wherein all or part of the recovered benzene is used as a component of the stream (a.1) or (a.1.1).

## Revendications

1. Procédé adiabatique exploité en continu pour la fabrication de nitrobenzène par nitration de benzène dans une unité de nitration, selon lequel
a) un courant contenant du benzène (a.1 1), qui comprend au moins 90 % en masse de benzène, par rapport à la masse totale de (a.1), est mis en réaction dans un réacteur avec un mélange d'acide sulfurique (a.2) et d'acide nitrique (a.3) en conditions adiabatiques, le benzène étant utilisé en un excès stoechiométrique par rapport à l'acide nitrique (a.3),
b) le produit de procédé obtenu à l'étape a) est séparé dans un appareil de séparation de phases en une phase aqueuse comprenant de l'acide sulfurique (b.1) et une phase organique comprenant du nitrobenzène (b.2),
c) la phase aqueuse (b.1) obtenue à l'étape b) est concentrée par évaporation d'eau en une phase aqueuse (c.1) présentant une concentration en acide sulfurique plus élevée par rapport à (b.1), la phase (c.1) étant recyclé en partie à en totalité dans l'étape a) et utilisée en tant que constituant de (a.2),
d) la phase organique (b.2) obtenue à l'étape b) est transformée en nitrobenzène pur (d.1), un courant contenant du benzène (d.2) étant obtenu, qui est utilisé en partie à en totalité en tant que constituant de (a.1) dans l'unité de nitration de l'étape a),
**caractérisé en ce que**
des composés organiques aliphatiques sont déchargés du procédé de manière ciblée à au moins un emplacement, de sorte qu'uniquement un courant contenant du benzène (a.1) qui présente une teneur en composés organiques aliphatiques inférieure à 1,5 % en masse, par rapport à la masse totale de (a.1), soit introduit dans le réacteur.

2. Procédé adiabatique pour la fabrication de nitrobenzène par nitration de benzène dans deux unités de nitration raccordées en série (100, 200), comprenant chacune au moins un réacteur (1, 1*), selon lequel
a) exclusivement du benzène (a.1.1) qui présente une teneur en composés organiques aliphatiques inférieure à 1,5 % en masse, par rapport à la masse totale de (a. 1.1), est mis en réaction avec un mélange d'acide sulfurique (a.2.1) et d'acide nitrique (a.3.1) en conditions adiabatiques dans le réacteur (1) de la première unité de nitration exploitée en continu (100), le benzène étant utilisé en un excès stoechiométrique par rapport à l'acide nitrique (a.3.1);
b) le produit de procédé obtenu à l'étape a) est séparé dans un appareil de séparation de phases (2) en une phase aqueuse comprenant de l'acide sulfurique (b. 1.1) et une phase organique comprenant du nitrobenzène (b.2.1),
c) la phase aqueuse (b.1.1) obtenue à l'étape b) est concentrée par évaporation d'eau en une phase aqueuse (c.1.1) présentant une concentration en acide sulfurique plus élevée par rapport à (b.1.1), la phase (c.1.1) étant recyclé en partie à en totalité dans l'étape a) et utilisée en tant que constituant de (a.2.1),
d) la phase organique (b.2.1) obtenue à l'étape b) est transformée en nitrobenzène pur (d.1.1), un courant contenant du benzène (d.2.1) étant obtenu,
et selon lequel le courant contenant du benzène (d.2.1) est mis en réaction dans la seconde unité de nitration exploitée de manière continue ou discontinue (200) dans un réacteur (1*) avec un mélange d'acide sulfurique (a.2.2) et d'acide nitrique (a.3.2) en conditions adiabatiques pour former du nitrobenzène, le benzène étant utilisé en un excès stoechiométrique par rapport à l'acide nitrique (a.3.2) de préférence de 2,0 % à 20 %, de manière particulièrement préférée de 5,0 % à 10 % de la théorie, puis le nitrobenzène ainsi formé étant transformé en nitrobenzène pur.

3. Procédé selon la revendication 1 ou 2, selon lequel les composés organiques aliphatiques sont choisis dans le groupe constitué par le cyclohexane, l'heptane, le méthylcyclohexane, le bicycloheptane, les isomères de diméthylcyclopentane, l'éthylcyclopentane, le pentane, le cyclopentane et le 2-méthylhexane.

4. Procédé selon l'une quelconque des revendications 1 à 3, selon lequel la pression mesurée par rapport à la pression atmosphérique dans la phase gazeuse de l'appareil de séparation de phases utilisé à l'étape b) est ajustée à une valeur de 20 mbar à 200 mbar, et selon lequel la température dans l'appareil de séparation de phases utilisé à l'étape b) est ajustée à une valeur de 100 °C à 140 °C, et selon lequel des composés organiques aliphatiques sont déchargés avec la phase gazeuse de l'appareil de séparation de phases utilisé à l'étape b).

5. Procédé selon la revendication 4, selon lequel un gaz inerte est introduit dans l'appareil de séparation de phases pour favoriser le déchargement des composés organiques aliphatiques, avec lequel des composés organiques aliphatiques sont déchargés par une conduite de gaz d'échappement raccordé à l'espace de gaz de l'appareil de séparation de phases.

6. Procédé selon l'une quelconque des revendications 2 à 5, selon lequel le produit de procédé obtenu lors de la réaction adiabatique dans le réacteur (1*) est séparé dans un appareil de séparation de phases (2*) en une phase aqueuse comprenant du soufre (b.1.2) et une phase organique comprenant du nitrobenzène (b.2.2), et la phase organique (b.2.2) = (15*) est débarrassée dans une colonne de distillation (7*) des composés organiques aliphatiques et du benzène en excès, puis mélangée avec la phase organique (b.2.1) = (15) de la première unité de nitration (100).

7. Procédé selon l'une quelconque des revendications 1 et 3 à 6, selon lequel les composés organiques aliphatiques déchargés sont traités par distillation en partie à en totalité pour récupérer le benzène déchargé conjointement avec les composés organiques aliphatiques.

8. Procédé selon la revendication 7, selon lequel le benzène récupéré est utilisé en partie à en totalité en tant que constituant du courant (a.1) ou (a.1.1).
